# EUROPEAN PATENT APPLICATION

(11) **EP 2 491 858 A1**
(43) Date of publication of application: **29.08.2012**
(21) Application number: 11156004.1
(22) Date of filing: 25.02.2011
(51) Int. Cl.: A61B 5/055, G01R 33/567

(54) **Magnetic resonance tomography (MRT) apparatus and method of operating a magnetic resonance (MR) apparatus**

(71) Applicant: MAX-DELBRÜCK-CENTRUM FÜR MOLEKULARE MEDIZIN, 13125 Berlin (DE)
(72) Inventor: Frauenrath, Tobias, 41836 Hückelhoven (DE); Niendorf, Thoralf, 52074 Aachen (DE)
(74) Representative: Lange, Sven

(57) **Abstract**

A magnetic resonance tomography (MRT) apparatus (1) for the examination of a body (14) comprises parameter acquisition devices (13) for the acquisition of cardiovascular parameters of the body (14) and a control device (15) in communication with the parameter acquisition devices (13) for synchronizing the imaging, wherein the control device (15) is adapted to analyse the data of at least two parameter acquisition devices (13) and to output a control signal based on the analysis.

## Description

The present invention relates in general to a magnetic resonance tomography (MRT) apparatus, a method of operating a magnetic resonance (MR) apparatus and the use of an MRT apparatus.

A magnetic field is a force that is created by a magnet or by moving electric charges. A magnetic field can for example be used for Magnetic Resonance Imaging (MRI), which is a imaging technique used to visualize detailed internal structures. The MRI depends on the generation of strong and uniform magnetic fields. A major specification of the static field in MRI is that it has to be substantially homogeneous over a predetermined region, known in the art as the "diameter spherical imaging volume" or "dsv." Errors or variation of less than 20 parts per million peak-to-peak (or 10 parts per million rms) are typically required for the dsv.

MRI equipment has undergone a number of refinements since the introduction of the first closed cylindrical systems. In particular, improvements have occurred in quality/resolution of images through improved signal to noise ratios and introduction of high and ultra high field magnets. Improved resolution of images, in turn, has led to MRI being a modality of choice for an increasing number of specialists for both structural anatomical and functional human MRI imaging.

The basic components of a typical magnetic resonance system for producing images for human studies include a main magnet (usually a superconducting magnet which produces the substantially homogeneous magnetic field (the Bo-field) in the dsv), one or more sets of shim coils, a set of gradient coils, and one or more RF coils.

A number of differently designed magnetic resonance measurements or reconstruction methods are available. The terms "data acquisition" or "measurements" encompass all types of measurements. One or more two-dimensional or three-dimensional image data sets, multiple image data sets from different slices of the examination subject and data sets with and without weighting with regard to a specific parameter can be obtained as a result of the post-processing of the acquired raw data. Within the scope of the post-processing it can also be necessary to supplement the data acquired within the scope of one measurement with data of other measurements, or to have to draw on additional data from other measurements for post-processing in order to obtain one or more image data sets from the acquired data.

Usually, such a measurement is composed of partial measurements. The partial measurements are basically of the same design; only the strength of the phase-coding gradient or of the slice gradients is varied in a predetermined manner. The partial measurement can include one or more preparation modules in order to weight measurement signal corresponding to a physical parameter. For example, multiple preparation modules are required if multiple image data sets from different positions of the patient are acquired and a separate preparation is required for every single one of these image data sets (known as slices).

As the MRI is not done in real time, it is necessary to synchronise the imaging time dependent processes such as physiological motion. In the state of the art, it is desirable to obtain diagnostic quality ECG signals while a patient is being monitored in a MRI system. Electrocardiogram (ECG) signals are based on the surface potentials of the heart. The contraction of the heart muscle is produced by an electrical excitation, which is detected with the ECG apparatus. The ECG signal is a periodic signal and the different phases of the cardiac cycle are designated with letters. The conventional sequence is P-Q-R-S-T-U. The R-spike exhibits the greatest amplitude; it is therefore normally used as a reference point of the triggering. Current ECG with filtering on MRI systems only allows gating. However, triggering is also available. Such ECG gating provides information regarding what part of the cardiac cycle the heart is at for purposes of triggering an MRI image to be taken at the desired point in the cardiac cycle. In addition, ECG signal waveforms can be distorted when acquired in standard MRI Systems with a field strength of 1.5 T or higher ( for example 3T or 7.0 T).

In ECG-triggered data acquisition, a partial measurement is started after detection of the trigger signal. One or more data acquisitions can ensue in this partial measurement. These data acquisitions can belong to a single image data set, or multiple data acquisitions can belong to multiple image data sets, or multiple data acquisitions can respectively belong to a single image data set. The design and the sequence of corresponding measurements are hereby known and therefore require no detailed explanation herein.

The time span provided between two R-spikes is designated as an RR-interval. The partial measurement to be conducted in this interval includes not only the data acquisition as such but also, as described, preparation steps necessary for this in the form of magnetization preparation modules. In a known method for ECG -triggered implementation of a magnetic resonance measurement, a partial measurement is conducted after detection of a R-spike. After the end of the partial measurement, the control device of the magnetic resonance tomography apparatus communicates with the ECG device in order to be able to start the next partial measurement after displaying the next R-spike. Since the heart beat of a patient is subject to a certain variability, a safety interval from the end of the (last) partial measurement to the point in time of the expected next R-spike is typically observed (maintained) in order to be able to compensate for such fluctuations. The full RR-interval is accordingly not utilized; the RR interval beginning with this R-spike is not used to implement a partial measurement. Additionally to the above described technique (called prospective triggering) to reduce cardiac motion artefacts a retrospective gating technique can be applied. Here, the MRI acquisition and a simultaneous ECG recording takes place continuously, whereby the data is reorganized according to the cardiac cycle during image reconstruction.

Accordingly, there is currently no diagnostic quality ECG system that can be used in the MRI system. The primary reason that it is not easy to obtain adequate ECG quality on standard 3T or higher MRI systems is the magneto-hydrodynamics (MHD) flow voltages, which are due to the flow of blood in the static magnetic field of the MRI system. The MHD flow voltages can have the same spectral characteristics as the true heart polarization signals and are thus difficult to extract. These MHD flow voltages are due to the flow of blood, which is a conductor, in a direction perpendicular to the static magnetic field, or other magnetic fields, of the MRI system. In fact, blood vessels can experience a force on them due to blood flow in the static magnetic field of the MRI system. It can be difficult, if even possible, to separate the true ECG signal from the signals produced by the heart pushing the blood through the vessels around the heart, which is problematic for triggering and for the imaging in general.

It was an object of the invention to provide an apparatus and/or method which overcome the disadvantages of the aforementioned state of the art.

In a first aspect the invention is directed towards a magnetic resonance tomography (MRT) apparatus for the examination of a body including parameter acquisition devices for the acquisition of cardiovascular parameters of the body and a control device in communication with the parameter acquisition devices for synchronizing the imaging, wherein the control device is adapted to analyse the data of at least two parameter acquisition devices and to output a control signal based on the analysis.

The term MRT or MR apparatus encompasses magnetic resonance systems like magnetic resonance imaging (MRI) devices and nuclear magnetic resonance (NMR) devices. The term body includes a human body and a body of an animal. The parameter acquisition devices as well as the control device can be realised in hardware and/or in software. Cardiovascular parameters can be for example electrophysiological, hydrodynamic, mechanical and/or opto-acoustic properties of one or more components of the cardiovascular system. The synchronisation of the imaging can include the acquisition, analysis and/or reconstruction of MR data which can take place while and/or after the MR data is recorded. The synchronisation takes most likely place with regard to heart activity.

The analysis of at least two parameter acquisition devices or of the data or signals thereof prevents an accidental miss-detection of the synchronisation event. Blanking windows with a deliberate fixed can be turned into blanking windows with a variable length given by the analysis of at least to parameter acquisition devices. Even more blanking windows with a variable length can be omitted through the use of at least two parameter acquisition devices . A further advantage is the ability to deliver a precise control signal like a trigger or a time stamp even at changing heart rates. The parallel data evaluation increases the reliability and delivers more precise information about the just dominant cardiac phase. This leads to enhanced definition or image sharpness for example at cardiovascular imaging. Using a retrospective MRT-technique the present invention improves the data allocation or mapping of MR data to the matching cardiac cycle with more than one mesh point or supporting point per cardiac interval.

The combination of information gained from two or more physiological signals or values enhances the situational awareness. Even if only one physiological value like for example the cardiac cycle is monitored with two or more parameter acquisition devices delivering different aspects or factors the basis for decision-making is broadened.

The parameter acquisition devices may comprise an ECG, a pulse oximeter, an acoustic triggering unit, a magnetohydrodynamic measurement device, a measurement device for the degree of oxygen saturation, a measurement device for oxygenated/deoxygenated haemoglobin concentration, a contrast agent device and/or an MR data set. These examples of parameter acquisition devices or cardiovascular parameter acquisition devices allow for a broad acquisition or capture of cardiovascular parameters of the body. This gives a wide base for data analysis and image synchronisation which leads to enhanced imaging. A contrast agent changes the accessed parameters for example in dependence of how the agent is delivered. The MR data or data set includes intrinsic information which can be utilized like for example self-gating, phase changes, contrast edges. This information can be utilised to monitor or track movement for example of the heart valve which allows conclusions with regard to the cardiac cycle.

The MRT apparatus may comprise a respiration detector in communication with the control device. A respiration detector can be used as a further input additional to the (cardiovascular) parameter acquisition devices. The further input can enhance stability and reliability. In the following reference is made to the handling or analysis of parameter acquisition devices, the respiration detector and the data or signals thereof respectively should be included in the discussions.

The control device may comprise a parallel structure for analysing the data of parameter acquisition devices in parallel. The parallel structure allows a fast analysis and a real time comparison and/or evaluation of the different parameter acquisition devices and the data or signals thereof respectively. Alternatively, a single structure can be used. The structure has less complexity but takes longer to handle more parameter acquisition devices.

The control device may comprise a decision unit for selecting one or more parameter acquisition devices for the calculation of the control signal. If for example one data acquisition device it not suitable or less suitable than one or more of the others the decision unit can decide not to use this device or the data of this device. This allows optimisation of the imaging by choosing the best data sources. For example, the ECG signal can get distorted in the magnetic field due to the magnetohydrodynamic effect. This often produces a false trigger event approximately at the time of the T wave. At this phase of the cardiac cycle the wave of the pulse oximeter is present as well so that parallel detection and handling can avoid a T wave based trigger. The hardware for the sensors is already present in most clinical MR systems. On the other hand, one can decide to use all data inputs or traces to process and/or reconstruct different images. Then, the operator or a medical practitioner can compare the images and decide which image or images can be used. This decision can also be made automatically by a computer. In other words, the quality control or decision which synchronisation leads to the best image can be decided automatically or user controlled, during the acquisition, analysis and reconstruction or afterwards.

The control device may be adapted to generate a trigger signal, a blanking signal and/or a cardiac cycle or heart rate tracer based on the analysis. These are examples of different control signals generated by the control device. The control device can be part of the MR apparatus like for example a control computer or a software routine or it can be realised outside the MR apparatus. The control signal can be used for the control of the MR apparatus or the acquisition, analysis, processing and reconstruction of MR data and/or images.

In a second aspect the invention is directed towards a method for operating a magnetic resonance (MR) apparatus for the examination of a body, the method comprising:
- acquisition of cardiovascular parameters of the body;
- analysis of at least two parameters;
- acquisition, analysis, processing and/or reconstruction of MR data based on the analysis of cardiovascular parameters.

The same advantages and modifications as described above apply here as well. The method can preferably be used for imaging of structural and/or functional abilities of tissue and organs in a body. The parameters may be captured by parameter acquisition devices as described above. Cardiovascular parameters can be electrophysiological, hydrodynamic, mechanical and/or opto-acoustic properties of one or more components of the cardiovascular system. These parameters and further inputs like contrast agent parameters, respiration data and/or information inherent to MR data can be traces for the acquisition, analysis, selection, processing and/or reconstruction of MR data.

The magnetic resonance data acquisition may be synchronized based on the analysis. The analysis can be utilized to control parameters of the MR apparatus like the control of the magnetic field, the HF coil or the like to optimise the imaging.

A trigger signal, a blanking signal and/or a cardiac cycle/heart rate tracer may be generated based on the analysis. These signals can be utilised to improve the acquisition, analysis, processing and/or reconstruction of MR data.

Magnetic resonance data ordering or magnetic resonance data reconstruction may be synchronized or controlled based on the analysis. This includes the allocation or mapping of MR data to corresponding phases of the cardiac cycle based on the analysis or evaluation of the sensor or parameter data. Sometimes this is referred to as AUTO-RECON.

Magnetic resonance data reconstruction and magnetic resonance data quality may be adapted based on the analysis which is sometimes called AUTO-IMAGE QUALITY. According to this approach images are reconstructed based on traces or combinations of traces and from these images those data or images are selected which comprise the least or alternatively the most motion or movement artefacts.

Information derived from the analysis of different parameters may be combined. The combination includes all operations like for example comparison, addition, subtraction, minimum, maximum and/or average of two or more parameters. This can be seen, in a way, as a secondary analysis subsequent to the analysis of (raw) parameter data. This approach can be used to improve the operation of the MR apparatus and the parameter analysis as well.

In a third aspect, the inventions concerns the use of a MRT apparatus for magnetic resonance, wherein at least a first and a second parameter are used for an acquisition, analysis and/or reconstruction of MR data. With the preferred apparatus it is possible to use at least two physiological parameters for the acquisition, analysis and/or reconstruction of MR data, thereby enhancing the image quality. The analysis of at least two parameters prevent an accidental detection of a distortion of aphysiological signal which might influence the acquisition, analysis and/or reconstruction of MR data. The parameters are preferably properties of at least one component of the cardiovascular system, which is a part of the circulatory system. The circulatory system is an organ system that passes nutrients (such as amino acids, electrolytes and lymph), gases, hormones, blood cells, etc. to and from cells in the body. The main components of the human cardiovascular system are the heart, the veinous, and the arterial blood vessels. It includes: the pulmonary circulation, a loop through the lungs where blood is oxygenated; and the systemic circulation, a loop through the rest of the body to provide oxygenated blood. The preferred parameters reflect characteristics or physiological properties of a component of the cardiovascular system, preferably electrophysiological, hydrodynamic, mechanical and/or opto-acoustic properties, such as pulse, average arterial pressure, systolic pressure, diastolic pressure, arterial wave form, blood flow, systole duration, or diastole duration. The usage of at least two physiological parameter allows delivering a precise control signal like a trigger even at changing heart rates. The acquisition, analysis and/or reconstruction of at least one parameter in correlation with at least a second parameter is performed preferably automatically or manually. It can be done by a suitable software or hardware or by a user controlling the apparatus. This is advantageous, as experienced users can optimize or amend the analysis in relations to the result to be achieved.

In a preferred embodiment the apparatus is used for imaging of structural and/or functional abilities of tissue and organs in a body. With the apparatus it is possible to structurally and/or functionally analyze an organ or tissue. According to the invention, an organ is preferably a collection of tissues joined in structural unit to serve a common function, chosen from the group comprising blood, lungs, heart, blood vessels, esophagus, stomach, liver, gallbladder, pancreas, intestines, kidneys, bladder, muscles, brain, spinal canal, prostate, parts of the skeletal system or spleen. A tissue is in accordance with the invention preferably an aggregate of cells in an organism that have similar structure and function. The fundamental types of tissues in humans are epithelial, nerve, connective, muscle, and vascular tissues. The apparatus can be used to image the structure of the aforementioned organs and tissues in high quality without imaging artefacts. Furthermore, the apparatus can be used to analyze the functions of the organs and tissues too. The apparatus can provide three-dimensional analysis of global and regional functions of preferably organs with great accuracy and reproducibility, thereby enabling a reliable and quick diagnosis of abnormal conditions of e.g. an organ, preventing failure of it.

It is also preferred to use the apparatus for angiographic and cardiovascular imaging and/or spectroscopy. Angiography is preferably the imaging of flowing blood in the arteries (arteriography), veins (phlebography) or lymphatic vessels (lymphography) of the body. The intensity in these images is proportional to the velocity of the flow. There are four preferred types of angiography, time-of-flight, phase contrast angiography, non-contrast MRA which makes use of differences in the blood flow across the cardiac cycle and contrast enhanced angiography. With the apparatus it is possible to detect changes of vessels, such as changes in the lumen, diameter, contractibility, stiffness of a vessel and/or stenosis, thrombosis or embolism. It is also preferred that the apparatus is used in cardiovascular imaging and/or spectroscopy. The preferred embodiment acquires information about the heart as it is beating and creates moving images of the heart throughout its cycle of contraction and relaxation. This allows to display abnormalities in cardiac chamber contraction and to show abnormal patterns of blood flow in the heart and the great vessels. This allows better assessment of complex anatomic abnormalities than with other imaging techniques. The preferred apparatus has the capability to identify areas of the heart muscle that are not receiving adequate blood supply from the coronary arteries and can clearly identify areas of the muscle that have become damaged as a result of infarction. It is also preferred to use the preferred apparatus for diagnostic imaging procedure for evaluating specifically: global cardiac function and regional wall motion abnormalities, ischemic heart disease, myocardial wall thickening myocardial hypertrophy, coronary artery disease, right ventricular abnormalities, pericardial disease, cardiac tumors, valvular disease, thoracic aortic disease, pulmonary artery disease, and congenital heart disease before and after surgical repair.

The apparatus can be used for magnetic resonance imaging (MRI), which is an imaging technique used primarily in medical settings to produce high quality images of the inside of the human body. Magnetic resonance imaging is based on the absorption and emission of energy in the radio frequency range of the electromagnetic spectrum, in particular by producing images based on spatial variations in the phase and frequency of the radio frequency energy being absorbed and emitted by the imaged object.

The apparatus can be used for various imaging techniques for example: gradient-echo based imaging, spin-echo based imaging, single or multislice imaging, volume imaging (3D imaging), ,multi- oblique imaging, inversion recovery imaging or other MR imaging techniques. Moreover, the MR apparatus can be utilized for spectroscopy. Magnetic resonance spectroscopy (MRS) is a noninvasive technique that can be applied to measure the concentrations of chemical components within tissues. The technique is based on the same physical principles as magnetic resonance imaging (MRI). MRI and MRS have in common, that the emitted radiofrequency is based on the concentration and spatial position of nuclei, with the exception that MRI usually but necessarily detects the signal of free water while MRS detects the chemical composition of tissue, cells or any other material. The information produced by MRS is preferably displayed graphically as a spectrum with peaks consistent with the various chemicals detected. MRS can be performed as an adjunct to MRI. MR imaging can be performed resolve the chemical composition of tissue, cells or any other material.

The information produced by MRS is preferably displayed graphically as a spectrum with peaks consistent with the various chemicals detected. MRS can be performed as an adjunct to MRI. Using the preferred embodiment, it is possible to analyze disorders of metabolism, tumors and certain inflammatory and ischemic diseases. It can be used for early diagnostic detection procedure but also for measuring changes in a variety of enzyme deficiencies, mitochondrial abnormalities, dystrophies, inflammatory myopathies, and thyroid disease. In muscle these diseases include preferably phosphofructokinase deficiency, amyloglucosidase deficiency, Duchenne muscular dystrophy, Becker muscular dystrophy, dermatomyositis, polymyositis, inclusion body myositis, hypothyroidism, and congestive heart failure.

In a preferred embodiment, the apparatus can be used in combination with a MR contrast agent, in order to further increase the imaging quality. However, it is also preferred, that the contrast agent can be used by the control device to generate a trigger signal, a blanking signal and/or a cardiac cycle tracer. Therefore, the contrast agent can be used for imaging itself but also for generating a signal. Hence, the concentration, the point of application, the time of application, the kind of agent and the retention period can be varied to generate various imaging quality or signal. MR contrast agents have been classified based on their chemical properties, mechanism of action and their biological distribution. They can be divided into gadolinium-based chelated agents (GBCA) and non-GBCA. The non-GBCAs include reticuloendothelial (RE) agents and hepatobiliary (HPB) agents. The GBCAs and HPB agents are paramagnetic, while the RE agents are super-paramagnetic. Based on the biological distribution, GBCAs behave similar to iodinated contrast used in computed tomography (CT), by distributing into the extracellular compartment. The RE agents are accumulated in the reticuloendothelial system. In a human or animal body, the agent can take a different course depending on its intended functionality: intravascular (IV), extracellular (EC), or intracellular (IC). An intravascular agent by design stays in the circulatory system until it is removed by the kidneys. Extracellular agents travel through the circulatory system and pass into the extracellular fluid, but do not enter into the cells. Intracellular agents can enter into a cell. It is also preferred to use targeted agents, which accumulate in a specific tissue or bind to a specific chemical compound, enzyme, receptor etc. The cause of the accumulation is an affinity for a specific tissue, chemical compound, enzyme, receptor etc. The person skilled in arts knows that endogenous or exogenous MR contrast agents exist, which comprise various substances that enhance or diminish a signal deriving from a tissue or an organ. Preferred agents comprise iron oxide, gadolinium chelate, manganese compounds, water, water in combination with gelling agents such as carboxymethylcellulose, mannitol or polyethylene glycol, fluorine or a fluorine-containing compound or hyperpolarized gases. Preferred endogenous MR contrast agents comprise blood.

The accompanying drawings are included to provide a further understanding of embodiments. Other embodiments and many of the intended advantages will be readily appreciated as they become better understood by reference to the following detailed description. The elements of the drawings do not necessarily scale to each other. Like reference numbers designate corresponding similar parts.
Fig. 1 illustrates a schematic view of a magnetic resonance apparatus according to the invention.
Fig. 2 shows the allocation of MR data to a cardiovascular parameter.
Fig. 3 shows diagrams of an ECG trace, a curve of a pulse oximeter and a phonocardiogram.
Fig. 4 shows diagrams of cardiovascular parameters with triggering points according to the invention.

In the following detailed description, reference is made to the accompanying drawings which form a part hereof and in which are shown by way of illustration specific embodiments in which the invention may be practised. In this regard, directional terminology, such as "top" or "bottom" etc. is used with reference to the orientation of the Figure(s) being described. Because components of embodiments can be positioned in a number of different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the appended claims.

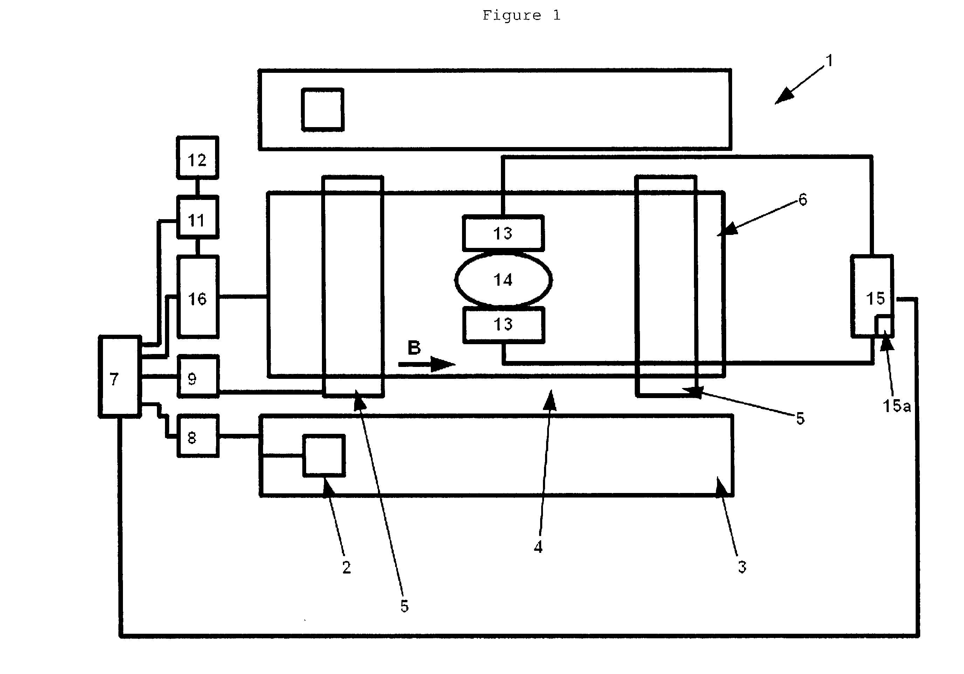

Figure 1 shows a magnetic resonance (MR) or magnetic resonance tomography (MRT) apparatus 1 like a magnetic resonance image (MRI) device or a nuclear magnetic resonance (NMR) device or any other device or system using magnetic resonance. The MR apparatus 1 has a generator 2 in form of magnets or coils which generate a basic magnetic field B. The generator 2 usually has a circular shape which is here shown in a sectional view. The generator 2 can comprise multiple magnets or coils, for the sake of clarity only one is shown. The generator 2 can be arranged in a housing 3 which can surround a tunnel shaped opening 4. Inside the opening or examination area 4 the magnetic field B is generated. The magnetic field B is directed through the opening 4 in direction of the arrow. The generator 2 is adapted to achieve a homogeneous field distribution.

The magnetic field apparatus 1 can include a second generator 5 which can be realized in form of time and spatially varying magnetic fields, which for example can be generated by additional coils, a specific version is commonly called gradient coil. The additional coils can work independently from the main magnetic field or their field can be superimposed to the main magnetic field. Only two additional coils 5 are depicted for the ease of understanding. It is common to use three additional coils, one for each direction but the number of additional coils is unlimited. The additional coils 5 can adapt or modulate the basic magnetic field B by generating gradient fields or they can generate a test field independent of the basic field B. The test field can be identical to the gradient fields. The second generator 5 can also be realised in form of one or more generators external to the housing 3.

The MR apparatus 1 includes a high frequency (HF) coil 6 to send HF excitation pulses and/or to detect the rotating transverse magnetisation of nuclear spins. The HF coil 6 is usually located inside the housing 3 near to the object to be examined.

The MR apparatus 10 includes a control unit 7 which controls the operation of the MR apparatus 10. The control unit 7 controls several subunits which can be internal or external to the control unit 7 and which can be implemented in hardware and/or software.

A main magnetic field control 8 controls the generator 2 and a gradient field control 9 controls the secondary generator 5. For the secondary generator 5 an external control i.e. independent from the MR apparatus 1 control can also be used.

HF control 10 includes an HF generator for exciting the HF coil 6 and the object 14 and therewith inducing transition of spins between energy levels which generates magnetic resonance signals when the spins return to the equilibrium. The HF coil 11 acts as a transmitter and/or as a receiver. The received MR signals are transmitted to an imager or spectrometer11. Here, the acquisition, analysis and/or reconstruction of MR data takes place. The imager 11 can be integrated into the control unit 7 and realised in hardware and/or software. The term acquisition can also encompass the whole process or part thereof of placing the object 14 inside the opening 4, generating the magnetic field or fields, exciting the HF coil 6 and the like.

The results or part of the results can be displayed at a display 12. The results can be an image of the object if an imaging process was chosen or it can be an analysis for example if a spectrography process was chosen.

The MR apparatus 1 further has parameter acquisition devices 13, two of which are exemplary depicted. More than two parameter acquisition devices 13 can be employed as well. The parameter acquisition devices 13 acquisition cardiovascular parameters of a body 14 which is arranged inside the opening or examination area 4 of the MR apparatus 1. A parameter acquisition device 13 can be an ECG, a pulse oximeter, an acoustic triggering unit, a magnetohydrodynamic measurement device, a measurement device for the degree of oxygen saturation, a measurement device for oxygenated/deoxygenated hemoglobin concentration, a contrast agent device and/or an MR data set. Cardiovascular parameters can be for example electrophysiological, hydrodynamic, mechanical and/or opto-acoustic properties of one or more components of the cardiovascular system. These parameters and further inputs like contrast agent parameters, respiratory information and/or information inherent to MR data can be traces for the acquisition, analysis, processing and/or reconstruction of MR data.

The parameter acquisition devices 13 are in communication with a control device 15. The control device 15 analyses the data of at least two parameter acquisition devices 13 and optionally of further information like for example from a respiration detector. The control device 15 generates and outputs a control signal based upon the analysis. The control device 15 is in communication with the control unit 7. The control device 15 can be implemented inside the control unit 7 as well. The control device 15 can be implemented in hard ware, soft ware or a combination of both.

The control device 15 is further adapted to record and/or detect signals from the acquisition devices 13 and from further inputs. These signals are evaluated or analysed inside the control device 15. The communication between the control device 15 and the MR apparatus 1 or more precisely the control unit 7 can be implemented in real time.

The control device 15 comprises a decision unit 15a for selecting one or more parameter acquisition devices 13 for the calculation of the control signal. The decision can depend for example on the condition of the MR apparatus 1, the desired imaging or how suitable the acquisition devices 13 or their signals are.

The control signal can for example be a trigger signal, a blanking signal and/or a cardiac cycle/heart rate tracer. It can further be a synchronisation signal or the signals can be utilised for synchronisation of the imaging.

The control signal can be utilized to acquisition, analyse, process, select and/or reconstruct MR data. An example for the acquisition is the control of the MR apparatus 1 as for example the control of the generator 2, the gradient coils 5 or the HF coil 6. An example for the analysis is to compare and/or choose the information or parameter acquisition device 13 which is suited best for the envisioned task. The MR data can be processed for example with a Fourier transformation in order to transform data from the frequency domain to images. The control signal can control the retrospective gating and the prospective triggering of the MR apparatus 1 based on the evaluation or analysis.

The steps of acquisition, analysis, processing and/or reconstruction as described above can be performed by the control device 15, the control unit 7, any external calculator or a combination thereof.

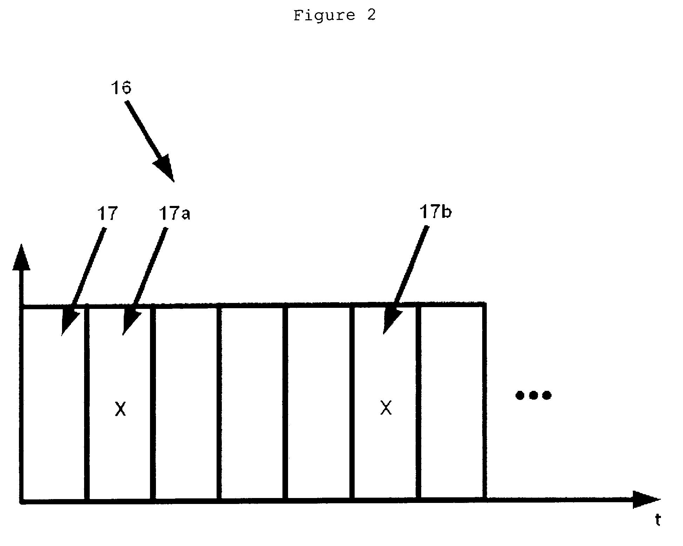

Figure 2 shows an example for the reconstruction of MR data 16. The raw or unallocated MR data 16 consists of a sequence of data slices 17 each of which can have a duration of approximately 20 ms. In the lower half of Figure 2 a signal behaviour of a physiological signal 18 is shown. The signal 18 can also be a combination of more than one physiological signal. The physiological signal 18 was received from one or more parameter acquisition devices 13. Here, an ECG trace is shown as an example.

The signal 18 can also be called a trace as it gives a marker or indication how to select data slices 17a and 17b from the sequence of data slices 17. Here, the R wave is the index. Accordingly, the data slices 17a, 17b, ... are selected to be reconstructed i.e. to be assembled to form an image. For example, about twelve data slices are put together. This process can also be described as a synchronisation of the imaging which means that MR data 16 for example in form of the shown slices 17 is synchronised with a parameter of the body like for example a physiological parameter or signal.

This magnetic resonance data reconstruction can also be referred to as magnetic resonance data ordering, both synchronized or controlled based on the analysis. Based on the analysis all aspects of the MR data handling can be influenced or improved based on the analysis. Another example would be that magnetic resonance data reconstruction and magnetic resonance data quality is adapted based on the analysis.

Fig. 3 shows diagrams of an ECG trace, a curve of a pulse oximeter and a phonocardiogram.

The various physiological parameters are detected at different time points of the cardiac interval. The acoustic triggering can be measured between the electrophysiological and the mechanical heart activity (approximately 30 ms time difference between the parameters).

The pulse wave of the pulse oximeter is delayed for approximately 300 ms after the heart activity and the measuring of the signal by the of the pulse oximeter. Furthermore, the maximum of the magnetohydrodynamic effect can be measured between the acoustic triggering and the pulse wave of the pulse oximeter.

Fig. 4 shows diagrams of cardiovascular parameters with triggering points according to the invention. A preferred embodiment combines the various physiological parameters for the synchronisation of MR imaging with cardiovascular properties or parameters. For example:

The ECG based detection of the R-spike can be suppressed when a pulse wave is measured by the pulse oximeter, in order to repress the magnetohydrodynamic (MHD) effect.

Furthermore, it is possible to measure the MHD effect e.g. with electrodes near the neck region, and in parallel measure the ECG signal. The ECG triggering can be adapted in correlation to the MHD effect, meaning that the ECG triggering preferably only takes place when the MHD effect is low. The preferred embodiment can also be used to optimize the acoustic triggering. In order to differentiate the first and second heart tone, the MHD effect, which usually takes place with the second heart tone, can be utilized in addition to the pulse oximeter signal. By blanking the measurement when the MHD effect or the pulse wave is the strongest, it is possible to properly distinguish between the different heart tones. The determination of the accurate heart phase enables a better and more reliable synchronisation, which ultimately improves the imaging quality of the MRT apparatus.

## Claims

1. Magnetic resonance tomography (MRT) apparatus for the examination of a body (14), comprising parameter acquisition devices (13) for the acquisition of cardiovascular parameters of the body (14), a control device (15) in communication with the parameter acquisition devices (13) for synchronizing the imaging, wherein the control device (15) is adapted to analyse the data of at least two parameter acquisition devices (13) and to output a control signal based on the analysis.

2. MRT apparatus according to claim 1, wherein the parameter acquisition devices (13) comprise an ECG, a pulse oximeter, an acoustic triggering unit, a magnetohydrodynamic measurement device, a measurement device for the degree of oxygen saturation, a measurement device for oxygenated/deoxygenated haemoglobin concentration, a contrast agent device and/or an MR data set (16).

3. MRT apparatus according to claim 1 or 2, comprising a respiration detector in communication with the control device (15).

4. MRT apparatus according to at least one of claims 1 to 3, wherein the control device (15) comprises a parallel structure for analysing the data of parameter acquisition devices (13) in parallel.

5. MRT apparatus according to at least one of claims 1 to 4, wherein the control device (15) comprises a decision unit (15a) for selecting one or more parameter acquisition devices (13) for the calculation of the control signal.

6. MRT apparatus according to at least one of claims 1 to 5, wherein the control device (15) is adapted to generate a trigger signal, a blanking signal and/or a cardiac cycle or heart rate tracer based on the analysis.

7. Method for operating a magnetic resonance (MR) apparatus (1) for the examination of a body (14), the method comprising:
- acquisition of cardiovascular parameters of the body (14);
- analysis of at least two parameters;
- acquisition, analysis, processing and/or reconstruction of MR data (16) based on the analysis of cardiovascular parameters.

8. Method according to claim 7, wherein magnetic resonance data acquisition is synchronized based on the analysis.

9. Method according to claim 7 or 8, wherein a trigger signal, a blanking signal and/or a cardiac cycle/heart rate tracer is generated based on the analysis.

10. Method according to at least one of claims 7 to 9, wherein magnetic resonance data ordering or magnetic resonance data reconstruction is synchronized or controlled based on the analysis.

11. Method according to at least one of claims 7 to 10, wherein magnetic resonance data reconstruction and magnetic resonance data quality is adapted based on the analysis.

12. Method according to at least one of claims 7 to 11, wherein information derived from the analysis of different parameters is combined.

13. Use of the apparatus according to claim 1 to 6 for magnetic resonance, wherein at least a first and a second parameter are used for an acquisition, analysis and/or reconstruction of MR data.

14. Use of an apparatus according to claim 13 for an automated or manual acquisition, analysis and/or reconstruction of at least one parameter in correlation with at least a second parameter, and/or for imaging of structural and/or functional abilities of tissue and organs in a body, and/or for angiographic and cardiovascular imaging and/or spectroscopy.

15. Use according to at least one of the preceding claims, wherein the parameter is an electrophysiological, hydrodynamic, mechanical and/or opto-acoustic property of one component of the cardiovascular system.

16. Use according to at least one of the preceding claims in combination with a MR contrast agent, whereby the contrast agent is preferably used by the control device to generate a trigger signal, a blanking signal and/or a cardiac cycle tracer.
